(19) Europäisches Patentamt
European Patent Office
Office européen des brevets

(11) Publication number: **0 421 636 A1**

# (12) EUROPEAN PATENT APPLICATION

(21) Application number: **90310275.4**

(22) Date of filing: **19.09.90**

(51) Int. Cl.[5]: **C12P 41/00**, C12P 11/00, C07D 207/16

(30) Priority: **26.09.89 US 412976**

(43) Date of publication of application: **10.04.91 Bulletin 91/15**

(84) Designated Contracting States: **AT BE CH DE DK ES FR GB GR IT LI LU NL SE**

(71) Applicant: **E.R. SQUIBB & SONS, INC.**
**Lawrenceville-Princeton Road**
**Princeton New Jersey 08543-4000(US)**

(72) Inventor: **Howell, Jeffrey M.**
**68 Ormont Road**
**Chatham, New Jersey(US)**
Inventor: **Patel, Ramesh N.**
**572 Cabot Hill Road**
**Bridgewater, New Jersey(US)**
Inventor: **Szarka, Laszlo J.**
**5 Wellington Road**
**East Brunswick, New Jersey(US)**

(74) Representative: **Thomas, Roger Tamlyn et al**
**D. Young & Co. 10 Staple Inn**
**London WC1V 7RD(GB)**

## (54) Enzymatic resolution process.

(57) S-Enantiomers of the formula

I

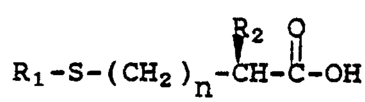

result from the reaction of a racemic form of compound I with an alcohol in the presence of an enzyme capable of selectively esterifying the R-enantiomers of the racemic compound.

EP 0 421 636 A1

## ENZYMATIC RESOLUTION PROCESS

In accordance with the present invention a novel process for preparing S-enantiomers having the formula

I 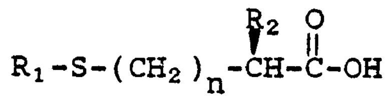

where $R_1$ is hydrogen or

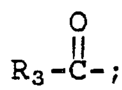

$R_2$ and $R_3$ are each independently selected from alkyl, cycloalkyl aralkyl or aryl; and n is 1 or 2, is provided. The present process comprises
reacting a racemic mixture of a compound having the formula

I' 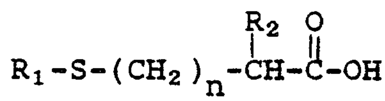

with a compound of the formula
VIII $R_4$-OH,
wherein $R_4$ is alkyl, cycloalkyl, aryl or aralkyl, in the presence of an enzyme or microorganism capable of catalyzing the selective esterification of compounds of formula I' to provide a solution containing R-enantiomeric esters and S-enantiomeric unreacted acids of formula I; and,
recovering the resulting non-esterified S-enantiomers of formula I.

### Detailed Description of Preferred Embodiments

The following definitions apply throughout this application.

The term "alkyl" as used herein refers to straight or branched chain carbon groups of 1 to 25 carbon atoms, preferably 1 to 6 carbon atoms.

The term "cycloalkyl" as used herein refers to groups containing 5 to 7 carbon atoms.

The term "aryl" as used herein refers to monocyclic or bicyclic aromatic groups containing from 6 to 10 carbon atoms in the ring portion such as phenyl, naphthyl, and substituted phenyl or naphthyl containing substituents such as nitro, halogen, methyl or alkoxy groups on the aromatic ring.

More specifically, it has been found that alcohols of formula VIII, in the presence of catalyzing lipases or esterases (or microorganisms producing same), are capable of catalyzing the stereoselective esterification of racemic compounds of formula I'. This process produces esters in the R-enantiomeric form having the formula

IX 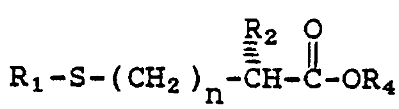

and the resulting "by-product" is in fact, a high yield of optically pure unreacted S-enantiomers of formula I.

The enzymatic resolution process of the present invention has the advantage that it can provide the desired S-enantiomers of formula I with high optical purity and at good yields. Additionally, because the present process uses a racemic carboxylic acid of formula I' as a starting material, instead of the carboxylic acid esters employed in prior art enzymatic processes, there is considerably less expense involved. These

and other features make the process of the present invention very attractive for use in preparing optically active compounds of formula I, such as the S-enantiomers of the formula

II 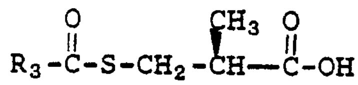 and

IIa 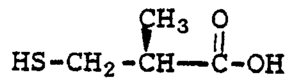

useful in the preparation of captopril and zofenopril.

Methods for obtaining the racemic starting material of the formula

I 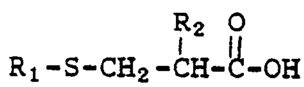

are known.

For example, a compound of the formula

VII $R_1$-SH

can be coupled to a compound of the formula

VIII 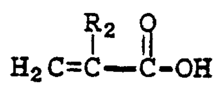

in the presence or absence of a suitable solvent, such as hexane, heptane or isopropanol, under the usual conditions for conducting such an addition reaction.

Compounds of formula

VIII $R_4$-OH

for use in the present process can be any alcohol suitable for esterifying compounds of formula $I'$. For example, in a preferred embodiment for providing resolved S-enantiomers of formula II and IIa, methanol, benzyl alcohol and 1-octanol each provided optical purities in excess of 95% at reaction yields of between about 30% and 40%.

The present process can be carried out in an organic solvent. Typical solvents suitable for use in the present process include, but are not limited to, 1,1,2-trichloro-1,2,2-trifluoroethane, toluene, cyclohexane, benzene, hexane, heptane, isooctane and octane.

The enzyme or microorganism used in the present process can be any enzyme or microorganism having the ability to catalyze the stereoselective esterification of acids of formula $I'$. Various enzymes, such as esterases and lipases, regardless of origin or purity, are suitable for use in the present invention. The enzyme can be in the form of a mixture of animal and plant enzyme, cells of microorganisms, crushed cells or extracts of cells.

Typical genuses of microorganism suitable as sources of catalyzing enzymes include *Mucor, Escherichia, Staphylococcus, Agrobacterium, Rhizopus, Aspergillus, Nocardia, Streptomyces, Trichoderma, Candida, Rhodotorula, Torylopsis, Bacillus, Alcaligenes, Pseudomonas, Brevebacterium, Enterobacter, Chomobacterium, Arthrobacter, Microbacterium, Mycobacterium, Saccharomyces, Penicillium, Botrytis, Chaetomium, Ophiobolus, Cladosporium* and the like.

Commercially available enzymes suitable for use in the present invention include lipases, such as Amano P (*Pseudomonas fluorescens*) which is preferred, Amano AY-30 (*Candida cylindracea*), Amano N (*Rhizopus niveus*), Amano R (*Penicillium sp.*), Amano FAP (*Rhizopus oryzae*), Amano AP-12 (*Aspergillus niger*), Amano MAP (*Mucor meihei*), Amano GC-4 (*Geotrichum candidum*), Sigma L-0382 (porcine pancreas), Sigma L-3001 (Wheat germ), Sigma L-1754 (*Candida cylindracea*), Sigma L-0763 (*Chromobacterium viscosum*) and Amano K-30 (*Aspergillus niger*). Additionally, enzymes derived from animal tissue include esterase from pig liver, α-chymotrypsin and pancreatin from pancreas.

Specific microorganisms suitable for use in the present process include *Pseudomonas fluorescens,*

*Pseudomonas putida, Pseudomonas ovalis, Escherichia coli, Staphylococcus aureus, Alcaligenes faecalis, Streptomyces griseus, Streptomyces clavuligervs, Nocardia erthropolis, Nocardia asteraides, Mycobacterium phlei, Agrobacterium radiobacter, Aspergillus niger, Rhizopus oryzae* and the like.

In the preferred process of the present invention, the alcohol of formula VIII and racemic starting material of formula I' are added to the desired organic solvent. The enzyme (or microorganism containing same) is added thereto. Typically, the enzyme is adsorbed onto a suitable carrier, e.g. diatomaceous earth (porous Celite Hyflo Supercel), microporous polypropylene (Enka Accurel® polypropylene powder), or a nonionic polymeric adsorbent such as Amberlite® XAD-2 (polystyrene) or XAD-7 (polyacrylate) from Rohm and Haas Co. This serves the purpose of immobilizing the enzyme which has the effects of controlling the enzyme particle size and preventing aggregation of the enzyme particles when used in an organic solvent. This can be accomplished, for example, by precipitating an aqueous solution of the enzyme with cold acetone in the presence of the Celite Hyflo Supercel followed by vacuum drying, or in case of nonionic polymeric adsorbent, incubating enzyme solutions with adsorbent on a shaker for desired time, removing excess solution and drying enzyme-adsorbent resins under vacuum. The reaction solution typically contains between about 5 and 250 mg of racemic starting material per ml of solvent. The enzyme added to the reaction solution may be present in concentrations ranging from about 5 to about 200 mg of enzyme per ml of solvent. While it is desirable to use the least amount of enzyme possible, the amount of enzyme required will vary depending upon the specific activity of the enzyme used.

When the reaction is conducted in an organic solvent, small amounts of water may be added to the reaction mixture. The water added to the reaction mixture may be present in concentrations ranging. from about 0.2 to about 100 mg of water per ml of solvent, or solvent saturated with water, and preferably is present in an amount of about 0.5-5 mg/ml. Incubation of the reaction solution can be at a temperature between about 4 and about 60 °C and is preferably carried out at about 30-50 °C. The reaction time can be appropriately varied depending upon the amount of enzyme used and its specific activity. Typical reaction times at 28 °C for optical purities of 90 percent and above are at least about 2 hours and can range up to about 96 hours for greater conversions and higher optical purities, e.g. optical purities exceeding 95 percent. Reaction times can be reduced significantly by increasing the reaction tempertaure and/or increasing the amount of enzyme added to the reaction solution. S-enantiomers of formula I can be isolated from the reaction mixture and purified by known methodologies such as extraction, distillation, crystallization, column chromatography, and the like.

As will be apparent to those skilled in the art, the process of the present invention can be carried out using microbial cells containing an enzyme having the ability to catalyze the stereo-selective esterification of compounds of formula I'. When using a microorganism to perform the resolution, the present process is conveniently carried out by adding the cells and the racemic starting material to the desired solvent. Cells may be used in the form of intact cells, dried cells such as lyophilized, spray-dried or heat-dried cells, immobilized cells, or cells treated with organic solvents such as acetone or toluene. Cells may also be used in the form of treated cell material such as ruptured cells or cell extract.

Using the methodology of U S. 4,105,776, the resolved acid of formula I or its chemical equivalent is used to acylate L-proline having the formula

VIII 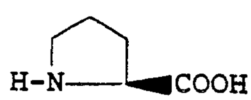 ,

forming captopril, i.e. the compound of formula III, in the case where $R_1$ in compound I is hydrogen. Preferably $R_1$ in compound I is an

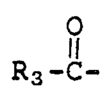

group, such as

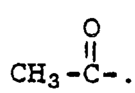

In this case, the thioester formed by the coupling of compound I with compound VIII can be deacylated by

conventional means, such as by ammonolysis (e.g., by treatment with alcoholic ammonia or concentrated ammonium hydroxide) or by alkaline hydrolysis (e.g., by treatment with aqueous metal hydroxide). Alternatively, again employing methodology from U. S. 4,105,776, the resolved acid of formula I can be used by removing the acetyl group (by conventional methods) and thereafter dehydrating the so-treated acid to form a thiolactone of the formula

IX 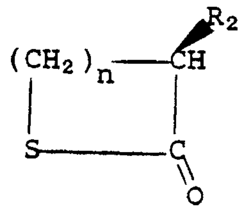

The thiolactone IX can thereafter be used to acylate the L-proline of formula VIII to obtain the desired product.

Similarly, to provide zofenopril, i.e., the compound of formula IV, the resolved acid of formula I or its chemical equivalent where $R_1$ is

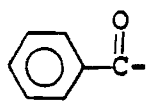

is used to acylate a compound of the formula

X 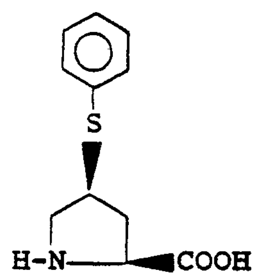

as described in U. S. 4,316,906. Alternatively, using the methodology of U. S. 4,316,906, the compound of formula I where $R_1$ is

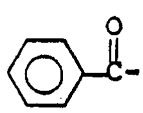

can be coupled with a compound of the formula

XI 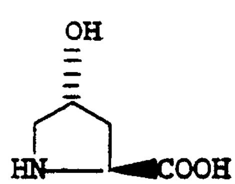 ,

or esters or protected forms thereof, to provide a compound of the formula

XII 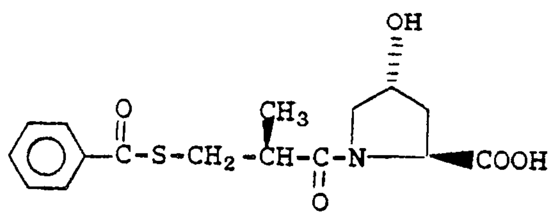

which can thereafter be treated with a compound of the formula

XIII 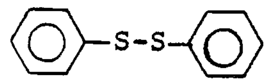

or

XIV 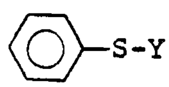

where Y is an activating group such as succinimide or phthalamido or a halide such as Cl or Br to provide the product of formula IV.

The acylation of compounds VIII, X or XI with the resolved acid of formula I can be effected in the presence of a coupling agent like dicyclohexycarbodiimide or the like, or the acid can be activated by formation of its mixed anhydride, symmetrical anhydride, acid chloride, acid ester or use of Woodward reagent K, N-ethoxy-carbonyl-2-ethoxy-1,2-dihydroquinoline or the like. For a review of the methods for acylation, see Methoden der Organischen Chemie (Houben-Weyl), Vol. XV, part II, page 1 et seq. (1974).

The present invention will now be described by the following examples, however, it should be understood that the invention is not meant to be limited by the details therein.

## Example 1

Racemic 3-benzoylthio-2-methylpropanoic acid (SQ 32,979; 6.726 g; 0.03 mol; 22.42 mg/ml) and methanol (3.845 g, 0.12 mol) were dissolved in and diluted with toluene to 300 ml. Crude Amano lipase P-30 (12 g; 40 mg/ml; *Pseudomonas fluorescens*) and deionized water (300 µl) were added and the reaction mixture was incubated at 28° C on a shaker at 280 RPM. The esterification was monitored by capillary gas chromatography (GC) with a flame ionization detector (FID) to determine reaction yields. At 198 hours 2.475 g of the remaining unreacted substrate, that is,
S(-)3-benzoylthio-2-methylpropanoic acid (SQ 25,680), had an optical purity of 97.1% with a reaction yield of 37% (results shown in Table 1). In this and all following examples, the enantiomeric composition of the unreacted 3-benzoylthio-2-methylpropanoic acid fraction was determined by capillary gas chromatography following derivatization with thionyl chloride and esterification of the resulting acid chloride with (S)-(+)-2-octanol to form diastereomeric esters which can be separated by capillary GC.

## Examples 2-4

Following the procedure of Example 1, additional resolutions were carried out with methanol, benzyl alcohol and 1-octanol. The results are summarized in Table 2.

Table 1

| Enzymatic Resolution of SQ 25,680 | | | | | |
|---|---|---|---|---|---|
| Reaction Time (hr) | Conversion (%) | Reaction Yield (%) | Optical Purity (%) | e.e. (%) | Enantiomeric Ratio E |
| 0 | 0 | 100 | 50.0 | 0.0 | |
| 136 | 57 | 43 | 92.3 | 84.6 | 12 |
| 159 | 58 | 42 | 95.0 | 90.0 | 15 |
| 166 | 59 | 41 | 95.5 | 91.0 | 14 |
| 198 | 63 | 37 | 97.1 | 94.2 | 12 |
| Reaction system: 0.1 M SQ 32,979 (22.42 mg/ml), 0.4 M methanol in 300-ml toluene; 300 uL (0.1 %v/v) deionized $H_2O$ added to reaction mixture. Enzyme: 12.0 g (40 mg/ml) Amano lipase P-30 (from Pseudomonas fluorescens) Conditions: 28° C, 280 rpm. | | | | | |

Table 2

| Ex. No. | Alcohol | Reaction Time (hr) | Conversion (%) | Reaction Yield (%) | Optical Purity (%) | e.e. (%) | Enantiomeric Ratio E |
|---|---|---|---|---|---|---|---|
| 2 | Methanol | 0 | 0.0 | 100.0 | 50.0 | 0.0 | |
| | | 16 | 5.8 | 94.2 | | | |
| | | 40 | 33.5 | 66.5 | 63.8 | 27.6 | 4.5 |
| | | 112 | 50.3 | 49.7 | 91.7 | 83.4 | 27.0 |
| | | 136 | 55.1 | 44.9 | 95.4 | 90.8 | 20.7 |
| | | 162 | 63.0 | 37.0 | 97.3 | 94.6 | 11.9 |
| | | 280 | 76.5 | 23.5 | 97.7 | 95.4 | 5.8 |
| | | 328 | 81.1 | 18.9 | 97.5 | 95.0 | 4.7 |
| 3 | Benzyl alcohol | 0 | 0.0 | 100.0 | 50.0 | 0.0 | |
| | | 16 | 10.9 | 89.1 | N.A. | | |
| | | 40 | 26.2 | 73.8 | 56.0 | 12.0 | 2.3 |
| | | 112 | 39.1 | 60.9 | 71.9 | 43.8 | 8.1 |
| | | 162 | 53.3 | 46.7 | 84.7 | 69.4 | 8.3 |
| | | 280 | 66.9 | 33.1 | 96.0 | 92.0 | 8.0 |
| | | 328 | 69.9 | 30.1 | 96.2 | 92.4 | 6.9 |
| 4 | 1-Octanol | 0 | 0.0 | 100.0 | 50.0 | 0.0 | |
| | | 16 | 9.8 | 90.2 | N.A. | | |
| | | 40 | 24.8 | 75.2 | 54.0 | 8.0 | 1.8 |
| | | 112 | 34.0 | 66.0 | 68.0 | 36.0 | 7.9 |
| | | 162 | 48.7 | 51.3 | 78.5 | 57.0 | 7.0 |
| | | 280 | 68.0 | 32.0 | 95.1 | 90.2 | 7.0 |
| | | 328 | 70.6 | 29.4 | 96.8 | 93.6 | 7.0 |
| Reaction system: 0.1 M SQ 32,979 (22.42 mg/ml), 0.4 M alcohol (indicated above) in 25 ml toluene; 25 uL (0.1 %v/v) deionized $H_2O$ added to reaction mixture. Enzyme: 1.0 g (40 mg/ml) Amano lipase P-30 (from Pseudomonas fluorescens). Conditions: 28° C, 280 rpm. | | | | | | | |

## Claims

1. A process for preparing S-enantiomers of the formula

I 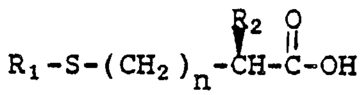

where $R_1$ is hydrogen or

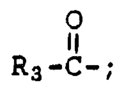

$R_2$ and $R_3$ are each independently selected from alkyl, cycloalkyl, aralkyl or aryl; and n is 1 or 2; which process comprises
reacting a racemic mixture of a compound having the formula

I' 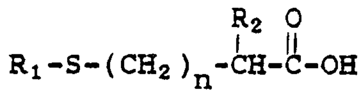

with a compound of the formula
VIII $R_4$-OH
wherein $R_1$ is alkyl, cycloalkyl, aryl or aralkyl, in the presence of an enzyme or microorganism capable of catalyzing the stereoselective esterification of compounds of formula I' to provide a solution containing R-enantiomeric esters and S-enantiomeric unreacted acids of formula I; and
separating and recovering the S-enantiomers of formula I from said solution.
2. The process o£ claim 1 wherein $R_4$-OH is selected from methanol, benzyl alcohol and 1-octanol.
3. The process of claim 1 wherein said enzyme is selected from esterases and lipases.
4. The process of claim 1 wherein the genuses of microorganisms are selected from *Mucor, Escherichia, Staphylococcus, Agrobacterium, Rhizopus, Aspergillus, Nocardia, Streptomyces, Trichoderma, Candida, Rhodotorula, Torulopsis, Bacillus, Alcaligenes, Pseudomonas, Brevebacterium, Enterobacter, Chromobacterium, Arthrobacter, Microbacterium, Mycobacterium, Saccharomyces, Penicillium, Botrytis, Chaetomium, Ophiobolus and Cladosporium.*
5. The process of claim 4 wherein said microorganisms are selected from *Pseudomonas fluorescens, Pseudomonas putida, Pseudomonas ovalis, Escherichia coli, Staphylococcus aureus, Alcaligenes faecalis, Streptomyces griseus, Streptomyces clavuligervs, Nocardia erthropolis, Nocardia asteraides, Mycobacterium phlei, Agrobacterium radiobacter, Aspergillus niger and Rhizopus oryzae.*
6. The process of claim 1 wherein said enzymes are selected from Amano P (*Pseudomonas fluorescens*) which is preferred, Amano AY-30 (*Candida cylindracea*), Amano N (*Rhizopus niveus*), Amano R (*Penicillium sp.*), Amano FAP (*Rhizopus oryzae*), Amano AP-12 (*Aspergillus niger*), Amano MAP (*Mucor meihei*), Amano GC-4 (*Geotrichum candidum*), Sigma L-0382 (porcine pancreas), Sigma L-3001 (Wheat germ), Sigma L-1754 (*Candida cylindracea*), Sigma L-0763 (*Chromobacterium viscosum*) and Amano K-30 (*Aspergillus niger*). Additionally, enzymes derived from animal tissue include esterase from pig liver, α-chymotrypsin and pancreatin from pancreas.
7. The process of any preceding claim wherein said reaction is in the presence of an organic solvent.
8. The process of claim 7 wherein said reaction is further in the presence of an amount of water of from about 0.2 mg to about 100 mg of water per milliliter of solvent.
9. The process of any preceding claim wherein the product of formula I is

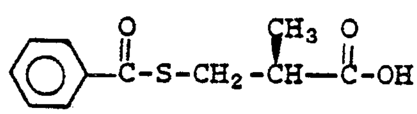 .

10. The process of any one of claims 1-8 wherein the product of formula I is

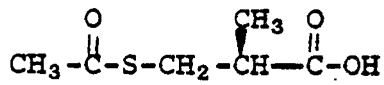 .

11. The process of any one of claims 1-8 wherein the product of formula I is

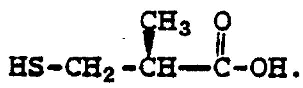

12. A process for the preparation of a compound of the formula

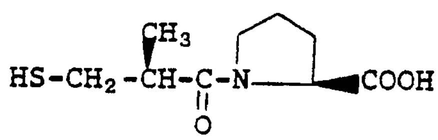

which comprises coupling a resolved carboxylic acid of the formula

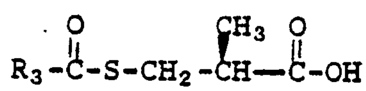

with a compound of the formula

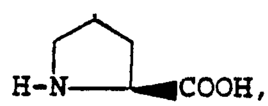

or its chemical equivalent,
and thereafter removing the

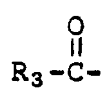

moiety to provide the desired product; wherein said resolved carboxylic acid is prepared by the process of any one of claims 1-8, $R_3$ being as defined in claim 1.

13. A process for the preparation of a compound of the formula

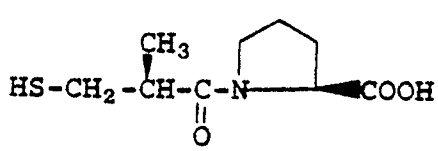

which comprises coupling a resolved carboxylic acid of the formula

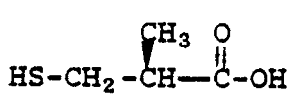

with a compound of the formula

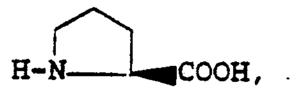

or its chemical equivalent,
wherein said resolved carboxylic acid is prepared by the process of any one of claims 1-8.
14. A process for the preparation of a compound of the formula

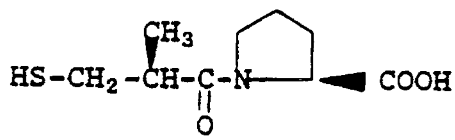

which comprises removing the acetyl group from a resolved carboxylic acid of the formula

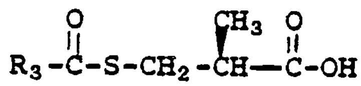

and dehydrating the so-treated acid to form a thiolactone of the formula

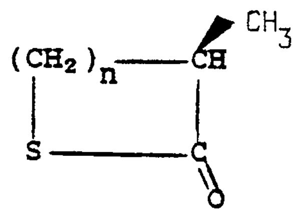

and thereafter acylating a compound of the formula

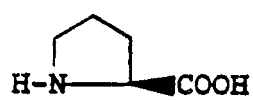

with said thiolactone to provide the desired product, wherein said resolved carboxylic acid is prepared by the process of any one of claims 1-8, $R_3$ being as defined in claim 1.
15. A process for the preparation of a compound of the formula

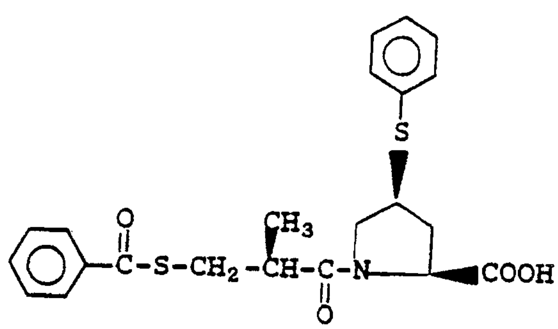

which comprises coupling a resolved carboxylic acid of the formula

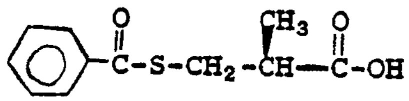

with a compound of the formula

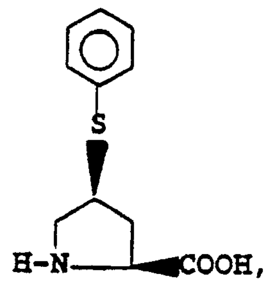

or its chemical equivalent,
which process is further characterized in that said resolved carboxylic acid is prepared by the process of any one of claims 1-8.

16. A process for the preparation of a compound of the formula

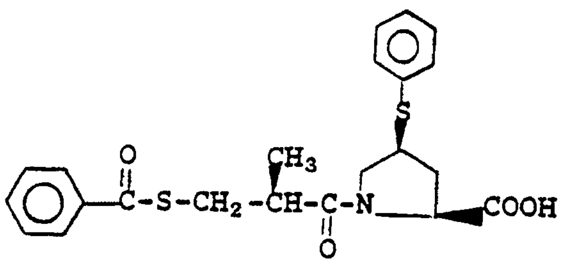

which comprises coupling a resolved carboxylic acid of the formula

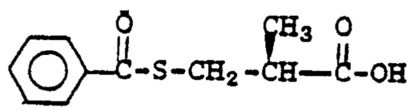

with a compound of the formula

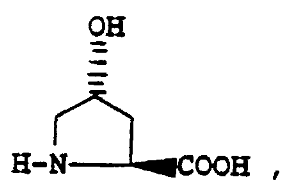

including esters or protected forms thereof,
and treating the so-formed intermediate with a suitable sulfide or disulfide to provide the desired product; which process is further characterized in that said resolved carboxylic acid is prepared by the process of any one of claims 1-8.

European Patent Office

# EUROPEAN SEARCH REPORT

Application Number

EP 90 31 0275

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN, vol. 13, no. 49 (C-565)[3397], 3rd February 1989; & JP-A-63 245 695 (SHOWA SHELL SEKIYU K.K.) 12-10-1988<br>* Abstract *<br>--- | 1-7 | C 12 P 41/00<br>C 12 P 11/00<br>C 07 D 207/16 |
| Y | WO-A-8 705 328 (WISCONSIN ALUMNI)<br>* Pages 1-5; claims *<br>--- | 1,12,13 | |
| Y | EP-A-0 172 614 (MITSUBISHI)<br>* Claims *<br>--- | 1,12,13 | |
| A | EP-A-0 130 752 (MITSUBISHI)<br>* Claims *<br>----- | 1 | |
| | | | TECHNICAL FIELDS SEARCHED (Int. Cl.5) |
| | | | C 12 P<br>C 07 D |

The present search report has been drawn up for all claims

| Place of search | Date of completion of the search | Examiner |
|---|---|---|
| THE HAGUE | 14-01-1991 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document